# EUROPEAN PATENT APPLICATION

(11) **EP 3 388 037 A1**
(43) Date of publication of application: **17.10.2018**
(21) Application number: 17166300.8
(22) Date of filing: 12.04.2017
(51) Int. Cl.: A61F 13/02, A61F 13/10

(54) **WEARABLE PRESSURE CARE DEVICE OR PAD**

(71) Applicant: Trulife Limited, Dublin (IE)
(72) Inventor: Murphy, Noel, Dublin, 6 (IE); Gunning, Olive, Summerhill Co Meath (IE)
(74) Representative: Tomkins & Co

(57) **Abstract**

The invention relates to wearable pressure care pads which can be applied to a patient's skin to provide pressure relief for that region. In particular, the pressure care pad is particularly suitable for protecting a patient's body proximities.

## Description

### Field of the Invention

The invention relates to wearable pressure care device or pad which can be applied to a region of patient's skin, or to a hard surface that the patient may come into contact with, to provide pressure relief for that region. In particular, the pressure care pad is particularly suitable for protecting a patient's body proximities.

### Background to the Invention

Many patients in hospitals and nursing homes are of reduced mobility because they may be bedridden, undergoing palliative care or are in a coma. Persons with muscle atrophy or spinal abnormalities may also be of reduced mobility because they are confined to a wheelchair. Such patients often develop pressure sores or pressure ulcers because they cannot move easily. Areas of high pressure can disrupt the flow of blood and the supply of oxygen to the skin which results in damage to the skin leading to sores and ultimately to ulcers. In addition patients with reduced mobility may also knock against a hard surface or object, resulting in bruises or abrasions in areas around their bony joints, such as the heel, the elbow or the wrist. These pressure sores, bruises or abrasions can cause considerable pain to a patient and can be distressing for both them and their families. The American Operating Room Nurses Association (AORN) has identified the need for specialised positioning equipment that will redistribute pressure, AORN (2013), Perioperative Standards and Recommended Practices.

There is also a need in some sports, such as weight lifting, soccer or rugby, to protect areas of the body, such as knees and elbows, from knocks or abrasions during practice or during matchplay.

### Object of the Invention

It is thus an object of the present invention to provide a pressure care tool that can be applied to the patient's body or to a hard surface to provide pressure relief for that region of the body, or to protect the patient if the patient knocks against the hard surface. A further object is that the pressure care tool is wearable. A still further object is that the pressure care tool has an adhesive surface so that it can be stuck on to the skin or a hard surface and will remain in place for a sustained period of time and that the adhesive is sufficiently sticky so that it may be removed from the skin or surface and replaced on the skin or surface, numerous times without losing its adhesive qualities. Still further objects include that the tool is durable, comfortable to wear on the skin, easy to use and easy to clean. Another object of the invention is that the pressure care tool is supple enough to move with the patient's body so that it is not restrictive of any movements that the patient can make, and so that it is comfortable to wear.

### Summary of the Invention

According to the present invention there is provided a pressure care tool comprising a first layer of silicone as a padding layer, the first layer having a layer of an adhesive silicone applied on one surface, and a layer of fabric or material positioned between the two silicone layers.

Preferably the pressure care pad is between 5 mm and 15 mm in thickness. Suitable thicknesses are between 8mm and 12mm.

In one embodiment, the layer of fabric or material extends through substantially all of the width of the pressure care pad. In an alternative embodiment the layer of fabric or material extends only through a portion of the width of the pressure care pad.

Suitable silicones for use in the first padding layer are multi-layer silicone networks. These may be silicone rubbers. One suitable material is a polydimethylsiloxine vinyl terminated 90% CAS number 68083-19-2 with 10% fumed silica CAS number 112945-52-5. Other similar silicones would be suitable for use as well. This layer forms a layer of pressure relief or padding in the pad.

Preferably the layer of adhesive is a silicone gel or another derivative of a multi-layer silicone network. Suitable materials are a polydimethylsiloxine vinyl terminated 85% CAS number 68083-19-2, or a methylhydrosiloxane copolymer 5% CAS number 68037-59-2 with 10% fumed silica CAS number 112945-52-5 respectively. These materials may be derivatives of the silicone used as the padding layer. Other similar silicones could be used.

The adhesive layer is tacky enough to remain on the body for a prolonged period of time without being easily knocked and rubbed off the skin. However it can be removed by gently pulling the pad off the skin of the patient. The adhesive layer is also sufficiently tacky that it can be replaced numerous times on the skin of the patient, without losing its adhesive qualities.

Preferably the fabric or material layer may be a layer of a natural or synthetic fabric, or it may be a woven or nonwoven fabric, but it may also be a layer of polyurethane. Other similar materials could be used.

In one embodiment of the invention the pad is formed in the shape of a star, with six arms. The free end of each arm is formed in a generally curved shape. Two of the arms are longer than the other four arms. This pad is particularly suitable for use as an elbow pad as the arms can be positioned in various ways around the joint, with or without overlap.

In an alternative embodiment of the invention the pad is formed with a paddle shaped body section having a stem shaped portion extending from a generally central portion of one side of the paddle. This embodiment is particularly useful for use as a heel pad.

In another embodiment of the invention, the pad is formed as an elongate element or strap. This embodiment is particularly suitable for use around the wrist or a around the ankle.

In a still further embodiment of the invention the pad is formed as a generally square or rectangular pad. This embodiment is particularly useful as a universal pressure care pad and can be used on large areas of the body such as the back or the buttocks, or on a hard surface.

However, the embodiments are not restricted to application to the skin. Depending on the user or on the surfaces into which a user may come in contact, the pressure of their care pad could be placed on a hard surface, such as a portion of a bed, a wall, a chair or other piece of furniture.

### Brief Description of the Drawings

- Figure 1: is an embodiment of the pressure care to formed as an elbow pad,
- Figure 2: is an embodiment of the pressure care to formed as a healer pad, and
- Figure 3: is an embodiment of the pressure care to formed as a universal positioner,
- Figure 4: is an embodiment of the pressure care pad formed as an elongate strap.
- Figure 5: is one embodiment of a mould for the production of the pressure pad, and
- Figure 6: is an alternative embodiment of a mould for the production of the pressure pad.

### Detailed Description of the Drawings

The pressure care pad (1) comprises a first layer of silicone (2) which acts as a padding layer or a pressure relieving layer. The first layer has an adhesive layer of silicone (3) on one surface. This layer also provides some padding effect. The adhesive layer is tacky enough to stick to the skin of the patient and to remain in situ on the patient's skin for a prolonged period of time. Between the silicone padding material and the adhesive layer is a fabric insert or a layer of material (4). The fabric insert is generally the same shape as the pressure care pad but need not extend throughout the entire body of the pad.

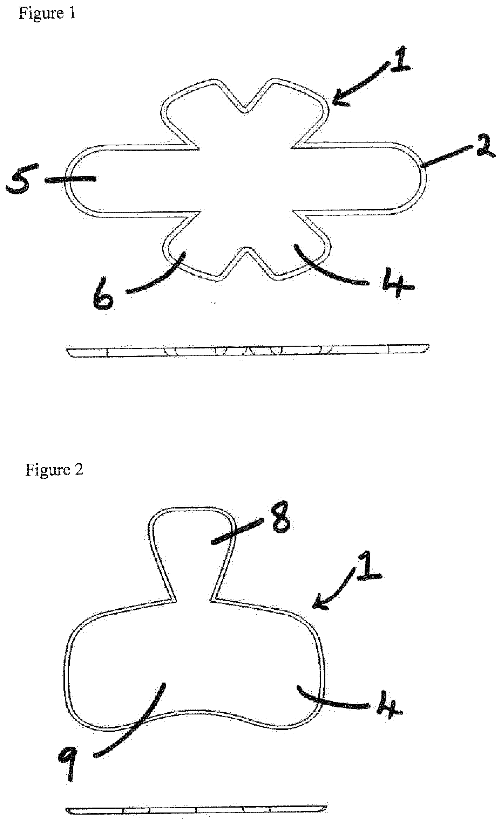

An embodiment of the pressure care pad which is suitable for use as an elbow pad, or on a hard surface, is shown in Figure 1. The pad (1) is formed from a layer of silicone (2) which is relatively thick compared to the layer of adhesive silicone (3) which is applied along one surface. Between the two layers is a layer of fabric (4). The pad is of a generally star shaped form with six arms. Two of the arms (5) are longer than the other four arms (6). These two longer arms (5) are opposed to each other across the shape of the pad. When the care pad is applied to the elbow, the arms (5,6) of the pad can be positioned on the skin so that there are no folds of silicone and it fits neatly around the elbow.

Figure 2 shows an embodiment of the pressure care suitable for use as a heel pad. As with the embodiment of Figure 1, the pad (1) is formed from a relatively thick first layer of silicone (2) which has a thinner layer of adhesive silicone (3) applied on one surface. A fabric layer (4) is positioned between the two layers of silicone roughly in the shape of the pad. The heel pad (1) is a roughly paddle shape (7) with a stem shaped portion (8) extending outwardly from approximately the central portion (9) of the paddle.

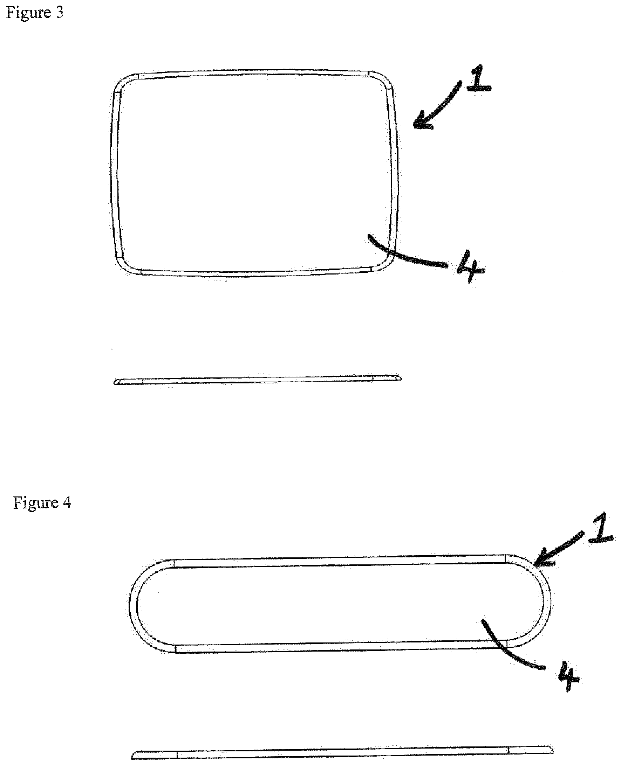

Figure 3 shows a universal pressure care pad (1) which is suitable for use on larger areas of skin such as the back, buttocks, the arms or legs. It is a generally square shape, but as the skilled person will appreciate, it could also be oblong. As with the embodiments shown in Figures 1 and 2, this pad (1) comprises a first thicker layer of silicone (2), a thinner layer of adhesive silicone (3) applied on one surface, with a layer of fabric (4) sandwiched between the two silicone layers. This embodiment is particularly suitable for use on hard surfaces.

As shown in Figure 4, the pressure care pad (1) can be formed as an elongate element or strap. As with previous embodiments, the strap is formed in three layers, a layer of silicone padding (2), a fabric layer (4), and a layer of adhesive silicone (3).

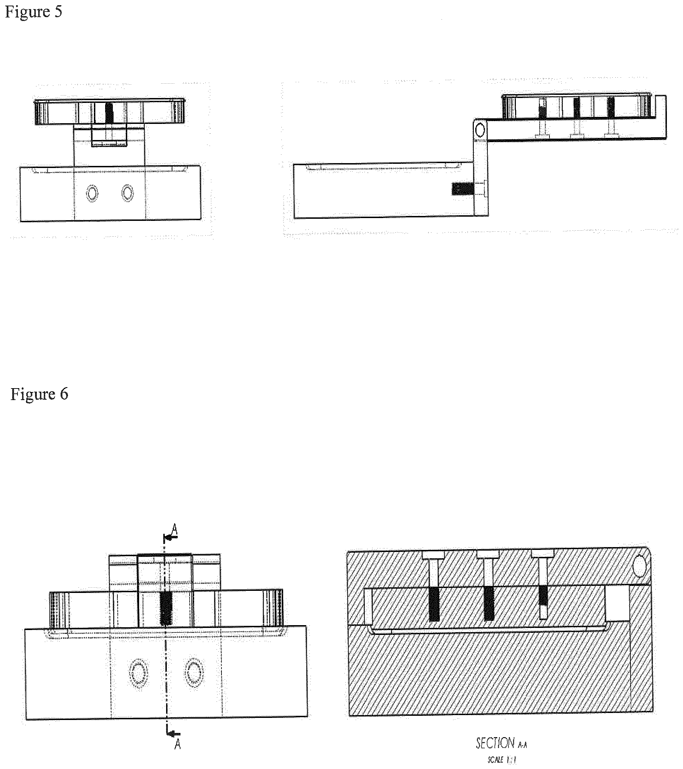

A method of producing a wearable pressure pad comprises pouring a first layer of silicone into an open mould, as shown in figure 5, with the silicone being in a non-cured state. The positive or male part of the mould is then placed on top of the silicone, and the silicone is allowed to cure. This creates a cavity in the product. The cavity can vary from 1.6mm to 3mm in depth depending on the thickness of the material being used in the product. Once the silicone has cured, the male part of the mould is removed leaving a cavity in one surface of the product. A layer of fabric material is then placed into the cavity. A layer of adhesive silicone is then dispensed into the cavity and onto the surface of the first silicone layer and allowed to cure.

The curing time for both the initial layer of silicone, and the layer of adhesive silicone, may be between 2 and 15 minutes, and generally takes place at a temperature of about 50 to 70°C.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A pressure care pad comprising a first layer of silicone as a padding layer, the first layer having a layer of an adhesive silicone applied on one surface, and a layer of fabric positioned between the two silicone layers.

2. A pressure care pad as claimed in claim 1 wherein the silicone for use in the first padding layer is a multi-layer silicone networks.

3. A pressure care pad as claimed in claim 2 wherein the silicone is a polydimethylsiloxine vinyl terminated 90% with 10% fumed silica CAS number 112945-52-5.

4. A pressure care pad the as claimed in claim 1 or claim 2 or claim 3 wherein the silicone for use in the layer of adhesive silicone is selected from a silicone gel or a derivative of a multi-layer silicone network.

5. A pressure care pad the as claimed in claim 4 wherein the adhesive silicone layer is a polydimethylsiloxine vinyl terminated 85% with 10% fumed silica, or a methylhydrosiloxane copolymer terminated 5% with 10% fumed silica.

6. A pressure care pad as claimed in any preceding claim wherein the fabric or material layer is a layer of a natural or synthetic fabric, a woven or nonwoven fabric, or a layer of polyurethane or other polymer material.

7. A pressure care pad as claimed in any preceding claim wherein the pad is formed in the shape of a star, with six arms.

8. A pressure care pad as claimed in claim 7 wherein the free end of each arm is formed in a generally curved shape.

9. A pressure care pad as claimed in claim 7 or claim 8 wherein two of the arms are longer than the other four arms.

10. A pressure care pad as claimed in any of claims 1 to 6 wherein the pad is formed with a paddle shaped body section having a stem shaped portion extending from a generally central portion on one side of the paddle.

11. A pressure care pad as claimed in any of claims 1 to 6 wherein the pad is formed as an elongate element or strap.

12. A pressure care pad as claimed in any of claims 1 to 6 wherein the pad is formed as a generally square or rectangular pad.

13. A pressure care pad as claimed in any preceding claim, wherein the layer of fabric on material extends throughout substantially all of the width of the pad.

14. A pressure care pad as claimed in any of claims 1 to 12, wherein the layer of fabric or material extends through a portion of the width of the pad.

15. A method of producing a wearable pressure pad comprising the steps:-
(a) pouring a first layer of silicone into an open mould, with the silicone being in a non-cured state,
(b) placing a male part of the mould on top of the silicone, and allowing the silicone to cure,
(c) removing the male part of the mould from the silicone leaving a cavity in one surface of the product,
(d) placing a layer of fabric material into the cavity, and
(e) dispensing a layer of adhesive silicone into the cavity and onto the surface of the first silicone layer and allowed it to cure.
